# EUROPÄISCHE PATENTSCHRIFT

(11) **EP 2 010 672 B1**
(45) Veröffentlichungstag und Bekanntmachung des Hinweises auf die Patenterteilung: **09.03.2011**
(21) Anmeldenummer: 07722247.9
(22) Anmeldetag: 18.04.2007
(51) Int. Cl.: C12Q 1/68, C12N 15/00

(54) **FORMULIERUNGEN UND VERFAHREN ZUR ISOLIERUNG VON NUKLEINSÄUREN AUS BELIEBIGEN KOMPLEXEN AUSGANGSMATERIALIEN UND NACHFOLGENDE KOMPLEXE GENANALYTIK**
FORMULATIONS AND METHOD FOR ISOLATING NUCLEIC ACIDS FROM COMPLEX STARTING MATERIALS AND SUBSEQUENT COMPLEX GENETIC ANALYSIS
FORMULATIONS ET PROCÉDÉ D'ISOLEMENT D'ACIDES NUCLÉIQUES CONTENUS DANS DES MATIÈRES PREMIÈRES COMPLEXES QUELCONQUES ET ANALYSE GÉNÉTIQUE COMPLEXE CONSÉCUTIVE

(30) Priorität: 25.04.2006 DE 102006019650
(43) Veröffentlichungstag der Anmeldung: 07.01.2009
(73) Patentinhaber: Invitek Gesellschaft Für Biotechnik & Biodesign MBH, 13125 Berlin (DE)
(72) Erfinder: BENDZKO, Peter, 12623 Berlin (DE); JOOS, Hans, 13507 Berlin (DE)
(74) Vertreter: Baumbach, Friedrich
(86) Internationale Anmeldenummer: PCT/DE2007/000689
(87) Internationale Veröffentlichungsnummer: WO 2007/121717

(56) Entgegenhaltungen:
- WO-A-00/34463
- WO-A-03/046178
- WO-A-2004/042058
- WO-A-2005/021748
- WO-A-2007/065934
- US-A1- 2007 042 384

## Beschreibung

Gegenstand der Erfindung sind Formulierungen ohne chaotrope Bestandteile zur Isolierung von Nukleinsäuren unter Bindung an eine feste Phase, insbesondere von DNA aus beliebigen komplexen Ausgangsmaterialien und Mengen enthaltend ein Lyse/Bindungspuffersystem, das mindestens eine antichaotrope Salzkomponente aufweist, eine feste Phase und an sich bekannte Wasch- und Elutionspuffer. Das Lyse/Bindungspuffersystem kann als wässerige Lösung vorliegen oder als feste Formulierung in einsatzfertigen Reaktionsgefäßen. Als feste Phase können alle Trägermaterialien fungieren, die zur Isolierung mittels chaotroper Reagenzien Anwendung finden, vorzugsweise Glasfaservliese, Glasmembranen, Siliciumträger Keramiken, Zeolithe oder Materialien, die negativ funktionalisierte Oberflächen besitzen oder chemisch modifizierte Oberflächen aufweisen, die in ein negatives Ladungspotential überführt werden können.

Gegenstand der Erfindung ist ferner ein Verfahren zur Isolierung von Nukleinsäuren, insbesondere von DNA, aus beliebigen komplexen Ausgangsmaterialien unter Verwendung der erfindungsgemäßen Formulierungen, das durch Lyse des Ausgangsmaterial, Bindung der Nukleinsäuren an ein Trägermaterial, Waschung der am Träger gebundenen Nukleinsäuren und Elution der Nukleinsäuren gekennzeichnet ist, wobei ggf. die nachfolgende Amplifikation von ausgewählten Sequenzabschnitten und ggf. eine nachfolgende Analyse der vervielfältigten Genabschnitte in ein und der selben Reaktionskavität durchgeführt werden kann. Die Anwendungsgebiete der Verfahren sind alle mit DNA-Isolierungen sich beschäftigenden Laboratorien, wie forensische Medizin, Lebensmitteldiagnostik, medizinische Diagnostik, Molekularbiologie, Biochemie, Gentechnik und alle anderen angrenzenden Gebiete.

Unter klassischen Bedingungen erfolgt die Isolierung von DNA aus Zellen und Geweben dadurch, dass die Nukleinsäuren enthaltenden Ausgangsmaterialien unter stark denaturierenden und reduzierenden Bedingungen, teilweise auch unter Verwendung von proteinabbauenden Enzymen aufgeschlossen, die austretenden Nukleinsäurefraktionen über Phenol-/Chloroform-Extraktionsschritte gereinigt und die Nukleinsäuren mittels Dialyse oder Ethanolpräzipitation aus der wässrigen Phase gewonnen werden (Sambrook, J., Fritsch, E.F. und Maniatis, T., 1989, CSH, "Molecular Cloning").

Diese "klassischen Verfahren" zur Isolierung von Nukleinsäuren aus Zellen und besonders aus Geweben sind sehr zeitaufwendig (teilweise länger als 48 h ), erfordern einen erheblichen apparativen Aufwand und sind darüber hinaus auch nicht unter Feldbedingungen realisierbar. Außerdem sind solche Methoden auf Grund der verwendeten Chemikalien wie Phenol und Chloroform in einem nicht geringen Maße gesundheitsgefährdend.

Verschiedene alternative Verfahren zur Isolierung von Nukleinsäuren aus unterschiedlichen biologischen Ausgangsmaterialien ermöglichen die aufwendige und gesundheitsschädigende Phenol-/Chloroform-Extraktion von Nukleinsäuren zu umgehen sowie eine Reduzierung der zeitlichen Aufwendungen zu erreichen.

Alle diese Verfahren basieren auf einer von Vogelstein und Gillespie (Proc. Natl. Acad. Sci. USA, 1979, 76, 615-619) entwickelten und erstmals beschriebenen Methode zur präparativen und analytischen Reinigung von DNA-Fragmenten aus Agarosegelen. Die Methode kombiniert die Auflösung der die zu isolierende DNA-Bande enthaltende Agarose in einer gesättigten Lösung eines chaotropen Salzes (NaJ) mit einer Bindung der DNA an Glaspartikel. Die an die Glaspartikel fixierte DNA wird anschließend mit einer Waschlösung (20 mM Tris HCl [pH 7,2]; 200mM NaCl; 2 mM EDTA; 50% v/v Ethanol) gewaschen und danach von den Trägerpartikeln abgelöst.

Diese Methode erfuhr bis heute eine Reihe von Modifikationen und wird zum gegenwärtigen Zeitpunkt für unterschiedliche Verfahren der Extraktion und Reinigung von Nukleinsäuren aus unterschiedlichen Herkünften angewendet (Marko, M.A., Chipperfield, R. und Birnboim, H.G., 1982, Anal. Biochem., 121, 382-387).

Darüber hinaus existieren heute weltweit auch eine Vielzahl von Reagenziensystemen, vor allem zur Reinigung von DNA-Fragmenten aus Agarosegelen und für die Isolierung von Plasmid DNA aus bakteriellen Lysaten, aber auch für die Isolierung von längerkettigen Nukleinsäuren (genomische DNA, zelluläre Gesamt-RNA) aus Blut, Geweben oder auch Zellkulturen.

Alle diese kommerziell verfügbaren Kits basieren auf dem hinlänglich bekannten Prinzip der Bindung von Nukleinsäuren an mineralische Träger unter Anwesenheit von Lösungen unterschiedlicher chaotroper Salze und verwenden als Trägermaterialien Suspensionen feingemahlener Glaspulver (z.B. Glasmilk , BIO 101, La Jolla, CA), Diatomenerden (Fa.Sigma) oder auch Silicagele. (Diagen, DE 41 39 664 A1).

Ein für eine Vielzahl unterschiedlicher Anwendungen praktikables Verfahren zur Isolierung von Nukleinsäuren ist in US 5,234,809 (Boom) dargestellt. Dort ist ein Verfahren zur Isolierung von Nukleinsäuren aus nukleinsäurehaltigen Ausgangsmaterialien durch die Inkubation des Ausgangsmaterials mit einem chaotropen Puffer und einer DNA-bindenden festen Phase beschrieben. Die chaotropen Puffer realisieren sowohl die Lyse des Ausgangsmaterials als auch die Bindung der Nukleinsäuren an die feste Phase. Das Verfahren ist gut geeignet, um Nukleinsäuren aus kleinen Probenmengen zu isolieren und findet speziell im Bereich der Isolierung viraler Nukleinsäuren seine praktische Anwendung.

Spezifische Modifikationen dieser Verfahren betreffen den Einsatz von neuartigen Trägermaterialien, welche für bestimmte Fragestellungen applikative Vorteile zeigen (Invitek GmbH WO-A 95/34569).

Entscheidende Nachteile von Verfahren der Isolierung von Nukleinsäuren aus komplexen Ausgangsmaterialien auf der Basis der Inkubation des Ausgangsmaterials mit einem chaotropen Puffer und einer festen Phase bestehen aber u.a. darin, dass der durch die chaotropen Puffer zu realisierende Zellaufschluss nicht für alle Materialien einsetzbar ist bzw. auch für größere Mengen an Ausgangsmaterialien nur extrem ineffizient und unter einem großen Zeitaufwand funktioniert. Darüber hinaus sind mechanische Homogenisierungsverfahren notwendig, wenn z.B. DNA aus Gewebeproben isoliert werden soll. Weiterhin müssen für verschiedene Fragestellungen auch immer verschieden hohe Konzentrationen unterschiedlicher chaotroper Puffer eingesetzt werden. Das Verfahren ist damit in keiner Weise universell einsetzbar.

Probleme, die durch eine ggf. schwierige Lyse des Ausgangsmaterials entstehen, können durch eine Reihe von kommerziell verfügbaren Produkten für die Nukleinsäureisolierung (speziell für die Isolierung genomischer DNA aus komplexen Ausgangsmaterialien) zwar gelöst werden, haben aber den großen Nachteil, dass es sich nicht mehr um ein klassisches "Single Tube"-Verfahren handelt, das das Verfahren gemäß US-Patent kennzeichnet, da die Lyse des Ausgangsmaterials in einem gebräuchlichen Puffer unter Einbeziehung eines proteolytischen Enzyms erfolgt. Die für die nachfolgende Bindung der Nukleinsäuren an z.B. Zentrifugationsmembranen notwendigen chaotropen Ionen müssen nach erfolgter Lyse dem Lyseansatz extra zugegeben werden. Sie können aber nicht Bestandteil des Lysepuffers sein, da die proteinzerstörende Funktion chaotroper Salze bekannt ist und natürlich sofort das für eine effiziente Lyse notwendige proteolytische Enzym ebenfalls zerstören würde.

Trotz einer Reihe von Nachteilen haben sich deshalb die Methoden der Nukleinsäureisolierung unter der Verwendung chaotroper Salze weltweit durchgesetzt und werden mittels kommerziell verfügbarer Produkte millionenfach eingesetzt. Diese Systeme sind extrem einfach in ihrer Durchführung und verfahren immer nach dem Prinzip der Lyse des Ausgangsmaterials, der nachfolgenden Bindung der Nukleinsäure an die feste Phase einer Glas-oder Silikamembran, welche sich in einem Zentrifugationssäulchen an einer Trägersuspension befindet, dem Waschen der gebundenen Nukleinsäuren und der nachfolgenden Elution der Nukleinsäuren mit einem Puffer geringer Ionenstärke.

Alle diese Systeme beruhen auf der Bindung der Nukleinsäuren an die jeweiligen Trägeroberflächen in Anwesenheit chaotroper Salze, d.h. mindestens eine Pufferlösung enthält als Hauptkomponente ein chaotropes Salz. Dies betrifft u.U. schon den Lysepuffer oder bei Systemen unter Einbeziehung proteolytischer Enzyme einen notwendigen Bindungspuffer, welcher nach der erfolgten Lyse des Ausgangsmaterials zugegeben wird.

Die Basis für chaotrope Salze sind die Reihen von Hofmeister zur Aussalzung von negativ geladenen, neutralen oder basischen Eiweißlösungen. Die chaotropen Salze sind dadurch charakterisiert, Proteine zu denaturieren, die Löslichkeit unpolarer Substanzen in Wasser zu erhöhen sowie hydrophobe Wechselwirkungen zu zerstören. Gerade diese Eigerischaften bewirken nach dem Stand der Technik auch mit Puffersystemen chaotroper Salze die übergeordnete Struktur des wässrigen Milieus zu zerstören um so die Bindung der Nukleinsäuren an ausgewählte feste Phasen zu vermitteln. Die bekanntesten Vertreter zur Nukleinsäureisolierung sind, Natriumperchlorat, Natriumjodid, Kaliumjodid, Guanidiniothiocyanat und Guanidinhydrochlorid. Sie sind jedoch zum einen kostenintensiv und zum anderen teilweise toxisch oder ätzend.

WO 2004/042058 (Invitek GmbH) offenbart Formulierungen von Puffern zur Isolierung, Reinigung und Rückgewinnung von lang- und kurzkettigen Nukleinsäuren, wobei die Nukleinsäure enthaltende Lösung mit Zusätzen so eingestellt wird, dass sie mono- und/oder multivalente Kationen sowie einen Alkohol und ggf. weitere Zusätze enthält. Dabei ist ein Mindesteinsatz an Pufferkonzentrationen von 10 mM beschrieben.

Eine Konzentration von antichaotropen Salzkomponenten unter 0,1 mM ist nicht offenbart.

Auf diesem Stand der Technik existieren bis zum heutigen Tag eine sehr große Anzahl an Patentanmeldungen sowie an erteilten Patente, wobei es sich dabei immer um Verfahrensvarianten handelt, wie z.B. die Verwendung neuer Trägermaterialien oder effizientere Waschpuffer etc., wobei das Grundprinzip immer die Verwendung chaotroper Salze zur Bindung an eine feste Phase aus Silicamaterialien darstellt.

Später wurde gefunden (EP 1 135 479, Inhaber: InViTek Gesellschaft für Biotechnologie & Biodesign mbH), dass eine Vielzahl ganz unterschiedlicher Salze als Bestandteile von ggf. auch an sich üblichen Lyse/Bindungspuffersystemen für die Bindung von Nukleinsäuren an klassische Trägermaterialien auf Basis von Glas oder Silica ausreichend sind. Die besten Ergebnisse konnten dabei mit Salzen erreicht werden, welche nach ihren chemisch-physikalischen Charakteristika die absolut entgegengesetzten Wirkungen in bezug auf die für die Nukleinsäurebindung bisher verwendeten chaotropen Salze aufweisen, Salze, die somit als antichaotrop bezeichnet werden können. So konnten mit Lyse/Bindungspuffern, deren Hauptkomponente z.B. Ammoniumsalze anstelle chaotroper Salze waren (kommerzielle Extraktionskits), bei Extraktionen genomischer DNA aus unterschiedlichen komplexen Ausgangsmaterialien (z.B. Blut, Gewebe, Pflanzen) unter Konstanz der bisher üblichen anderen Reaktionskomponenten, Trägermaterialien sowie bei völlig gleichem Reaktionsablauf mindestens dieselben quantitativen sowie qualitativen Resultate erreicht werden. Das bedeutet, dass mit einem Salz welches Proteine nicht denaturiert, sondern stabilisiert, welches die Löslichkeit unpolarer Substanzen in Wasser nicht erhöht, sondern absenkt sowie welches hydrophobe Wechselwirkungen nicht zerstört, sondern verstärkt, es genauso möglich ist, Nukleinsäuren, auch aus komplexen Ausgangsmaterialien zu isolieren, aufzureinigen und den an sich üblichen Applikationen zuzuführen. Antichaotrope Komponenten sind im vorliegenden Zusammenhang Ammonium-, Cäsium-, Natrium- und/oder Kaliumsalze, vorzugsweise Ammoniumchlorid. Gemäß EP 1 135 479 werden diese antichaotropen Salzkomponenten in Ionenstärken von 0,1 M bis 8 M eingesetzt.

Völlig überraschend wurde nun gefunden, dass auch bei deutlich niedrigeren Konzentrationen der antichaotropen Salzkomponenten die gewünschte Wirkung erzielt werden kann. Die vorliegende Erfindung betrifft dem gemäß Formulierungen und Verfahren ohne chaotrope Bestandteile zur Isolierung von Nukleinsäuren unter Bindung an eine feste Phase, insbesondere von DNA aus beliebigen komplexen Ausgangsmaterialien, die ein Lyse/Bindungspuffersystem enthalten, das mindestens eine antichaotrope Salzkomponente aufweist, wobei die Konzentration der antichaotropen Salzkomponente zwischen 0,1 mM und 1 mM beträgt, sowie ferner eine feste Phase und an sich bekannte Wasch- und Elutionspuffer.

Das Lyse/Bindungspuffersystem weist außerdem an sich bekannte Detergenzien und ggf. Zusatzstoffe auf, so z.B. Tris-HCl, EDTA, Polyvinylpyrrolidone, CTAB, TritonX-100, N-Lauryl-Sarkosin, Natriumcitrat, DTT, SDS und/oder Tween. In einer bevorzugten Ausführungsvariante enthält das Lyse/Bindungspuffersystem zur Bindung an die feste Phase einen Alkohol, wie z.B. Ethanol und Isopropanol und ggf. Enzyme, vorzugsweise Protein-abbauende Enzyme, z.B. eine Proteinase.

Die Erfindung ermöglicht somit über die Verwendung der neuartigen Kompositionen von Lyse/Bindungspuffern auf der Basis von antichaotropen Salzen in sehr geringer Konzentration für die Nukleinsäurenisolierung, speziell für die Isolierung genomischer DNA, basierend auf der Bindung der Nukleinsäuren an die an sich gebräuchlichen unterschiedlichen festen Phasen aus Silica bzw. Glasmaterialien, die Nutzung eines alternativen Chemismus als essentiellem Bestandteil von entsprechenden Testkits (Formulierungen).

Das erfindungsgemäße Verfahren unter Einbeziehung von antichaotropen Salzen folgt dabei den von der praktischen Laborroutine bekannten Verfahrensabläufen für die Nukleinsäureisolierung und ist charakterisiert durch:
1. Lyse des Ausgangsmaterials
2. Bindung der Nukleinsäuren an eine feste Phase
   (Zentrifugationssäule oder Suspension)
3.Waschen der gebundenen Nukleinsäuren
4. Elution der Nukleinsäuren mit einem an sich bekannten Niedrigsalzpuffer.

Die Erfindung ermöglicht eine hocheffiziente und schnelle Isolierung von Nukleinsäuren, besonders genomische DNA aus jedem beliebigen und ggf. komplexen Ausgangsmaterial. Die für die Bindung notwendigen antichaotropen Ionen können selbst bei Einbeziehung von proteolytischen Enzymen Bestandteil des Lyse/Bindungspuffers sein. Das erfindungsgemäße Verfahren ist damit einfach handhabbar und universell einsetzbar.

Die Realisierung der Nukleinsäureisolierung, insbesondere von DNA, aus beliebigen Ausgangsmaterialien erfolgt durch die Inkubation des die Nukleinsäure enthaltenden Ausgangsmaterials ohne Verwendung chaotroper Substanzen, die mit dem
- Lyse/Bindungspuffersystem, welches eine wässerige Lösung umfasst, die eine antichaotrope Salzkomponente, mindestens ein Detergenz, ggf. Zusatzstoffe und ggf. ein Enzym aufweist,
- und einer beliebigen festen Phase, vorzugsweise Glasfaservliese, Glasmembranen, Gläser, Zeolithe, Keramik sowie andere Siliciumträger in Kontakt gebracht werden,
wodurch die Lyse des Ausgangsmaterials und die nachfolgende Bindung der DNA an die feste Phase erfolgt. Anschließend wird die gebundene Nukleinsäure nach an sich bekannten Methoden gewaschen und die DNA von der festen Phase gelöst.

Bei bestimmten Extraktionsprotokollen kann der Lyseansatz ggf. mit einem zusätzlichen Detergenz, einem Alkohol oder einem Detergenz/Alkohol-Gemisch versetzt werden.

Bevorzugte Ausgangsmaterialien sind kompakte Pflanzenmaterialien, wie z.B. Früchte; Samen; Blätter; Nadeln etc., klinisch relevanten Proben, wie z.B. Vollblut; Gewebe, Mikrobioptate, paraffinierte Materialien, ercp-Proben, Tupfermaterial von Abstrichen, Lebensmittel, wie z.B. Fisch, Wurst, Konserven, Milch, forensischen Proben, wie z.B. Haarwurzeln, Zigarettenkippen, Blutspuren und andere Proben, die DNA enthalten.

Bevorzugte Ionen im Sinne der Erfindung sind die in der Hofmeister-Serie dargestellten antichaotropen Ammoniumionen, Cäsiumionen sowie Kalium- und Natriumionen oder Kombinationen dieser Ionen, vorzugsweise Ammoniumchlorid. Es können durch die Verwendung der antichaotropen Salze, die proteinstabilisierend wirken, als essentielle Bestandteile eines Lysepuffers in einer bevorzugten Ausführungsform der Erfindung auch proteolytische Enzyme, wie z.B Proteinase K, zur Unterstützung und Effektivierung des Lyseprozesses zugesetzt werden.

Puffersysteme des Standes der Technik mit den bekannten chaotropen Salze können bei den notwendigen hohen Ionenstärken, wie sie allgemein für eine quantitative Isolierung von Nukleinsäuren gefordert werden, keine proteolytischen Enzyme enthalten. Sie müssen somit immer nachträglich für die Bindung der Nukleinsäuren an die feste Phasen zugesetzt werden.

Als Detergentien in den erfindungsgemäßen Lysepuffem/Bindungspuffern werden bevorzugt anionische, kationische oder neutrale, wie z.B. SDS, Triton X-100, Tween oder CTAB eingesetzt.

Nach der erfolgten Lyse des Ausgangsmaterials wird die Suspension ggf. durch einen kurzen Zentrifugationsschritt von noch nicht vollständig lysierten Bestandteilen abgetrennt und mit dem DNA-bindenden Material direkt inkubiert bzw. wie schon beschrieben nach Zugabe mit einem zusätzlichen Detergenz, einem Alkohol oder einem Detergenz/Alkohol-Gemisch mit der festen Phase inkubiert. Gegebenenfalls befinden sich im Lysepuffersystem zusätzlich geringe Konzentrationen (< 50 mM) an EDTA und/oder Tris-HCl. Für die Isolierung von DNA aus sehr stark verunreinigten Ausgangsmaterialien erfolgt bevorzugt auch der Zusatz von 2-4% Polyvinylpyrrolidon oder anderen bekannten Substanzen zum Puffersystem zur selektiven Bindung von inhibitorischen Komponenten.

Als Bindungsmaterialien für die zu isolierende DNA haben sich z.B. kommerziell verfügbare Glasfaservliese in Zentrifugationssäulen, Siliziumverbindungen wie SiO₂ unterschiedlicher Teilchengröße hervorragend bewährt. Damit können alle die Materialien verwendet werden, welche für die Isolierung von Nukleinsäuren mittels chaotroper Puffer genutzt werden.

Nach der Inkubation mit dem DNA-bindenden Material erfolgt die Abtrennung des Lysates vom Bindungsmaterial durch einen kurzen Zentrifugationsschritt. Nachfolgend wird in an sich bekannter Weise mit einem Waschpuffer z.B. bestehend aus mindestens 50% Ethanol und gegebenenfalls einer geringen Salzkonzentration z.B. NaCl gewaschen, das Trägermaterial wird getrocknet und die gebundene DNA mittels eines an sich bekannten Niedrigsalzpuffers (Tris-HCl; TE; Wasser) und bei einer bevorzugten Temperatur von 50-70°C eluiert.

Eine weitere Ausführungsvariante der Erfindung besteht darin, dass zur Lyse von schwer aufschließbaren Ausgangsmaterialien, z.B. kompakten Gewebeproben, Haarwurzeln bzw. zur Optimierung der Lyseeffizienz und zur Reduzierung notwendiger Lysezeiten der Zusatz von proteolytischen Enzymen, vorzugsweise Proteinasen, wie z.B. Proteinase K, erfolgt.

Die Erfindung ermöglicht somit auf neuartigen Kombinationen antichaotroper Salze als essentiellen Bestandteilen von Lysepuffermixturen universell einsetzbare Verfahren zur Isolierung von Nukleinsäuren, insbesondere DNA, aus allen DNA enthaltenden Ausgangsmaterialien wie auch aus beliebigen Mengen an unterschiedlichsten Ausgangsmaterialien, wobei alle die bisher eingesetzten Trägermaterialien und deren Ausführungen genauso effizient eingesetzt werden können, wie auch die bisher praktizierten Vorschriften der Isolierung von Nukleinsäuren identisch nutzbar sind.

In seiner allgemeinsten Anwendungsvariante kann mittels des erfindungsgemäßen Verfahrens aus allen dem Stand der Technik entsprechenden für eine DNA-Extraktion ausgewählten komplexen Ausgangsmaterialien eine Nukleinsäureextraktion durchgeführt werden, d.h. mittels des neuen universellen Puffersystem kann die hocheffiziente Lyse und nachfolgende Nukleinsäurebindung an einen mineralischen Träger aus kompakten Pflanzenmaterialien (z.B. Früchte; Samen; Blätter; Nadeln etc.), aus klinisch relevanten Proben (z.B. Vollblut; Gewebe, Mikrobioptate, paraffinierte Materialien, ercp-proben, Tupfermaterial von Abstrichen), aus Lebensmitteln (z.B. Fisch, Wurst, Konserven, Milch), aus forensischen Proben (z.B. Haarwurzeln, Zigarettenkippen, Blutspuren) wie auch aus anderen Ausgangsmaterialien erfolgreich, extrem einfach und sehr schnell durchgeführt werden.

Ein weiterer Vorteil des Verfahrens besteht auch darin, dass die Isolierung von DNA dabei sowohl aus extrem geringen Ausgangsmaterialien (z.B. Isolierung von DNA aus 1 µl Vollblut; Haarwurzel, Mikrobiopsie < 1 mg) als auch aus sehr großen Mengen an Ausgangsmaterialien wie z.B. 50 ml Vollblut; 1 g Gewebematerial, <1g Pflanzenmaterial hocheffizient durchgeführt werden kann.

Neben einer allgemeinsten Ausführungsvariante erlauben Optimierungen des Extraktionsverfahrens bezogen auf spezifische Applikationen sogar eine fast quantitative Isolierung der in der Ausgangsprobe enthaltenen DNA-Mengen.
Das erfindungsgemäße Verfahren eignet sich auch hervorragend für die Konzipierung von automatisierbaren Systemen, bei welchen bekanntermaßen der Preis/Präparationen ein entscheidendes Auswahlkriterium ist.

Die erfindungsgemäßen Formulierungen ermöglichen in überraschender Weise den Zugang zu weiteren hochinteressanten und neuartigen Applikationen auf dem Gebiet der Nukleinsäureisolierung und Diagnostik.

In einer weiteren Ausführungsform der Erfindung sind die vorliegenden neuen Lyse/Bindungspuffersysteme, die mindestens eine antichaotrope Salzkomponente aufweisen, in der Lage Nukleinsäuren an feste Phasen zu binden, die eine negativ geladene Oberfläche besitzen oder Oberflächen, die ein negatives Ladungspotential aufweisen.

Aus dem Stand der Technik sind Verfahren und Mittel zu Nukleinsäurereinigung bekannt, wobei die Nukleinsäurebindung an chemisch modifizierte feste Phasen erfolgt (United States Patent: 5,523,392; Purification of DNA on Aluminium Silicates and Phosphosilicates; United States Patent: 5,503,816; Silicates Compounds for DNA Purification; United States Patent: 5,674,997; DNA purification on modified Siligates; United States Patent: 5,438,127; DNA Purification by solid phase extraction using a PCl₃ modified glass fiber membrane; United States Patent: 5,606,046: DNA purification by solid phase extraction using trifluormetric acid washed glass fiber membrane; United States Patent: DNA purification by solid phase extraction using glass fiber membrane previously treated with trifluoroacetic acid, and then with fluoride ion, hydroxyd ion, or BCL₃; United States Patent: 5,610,291: Glass fiber membranes modified by treatment with SiCl₃, AlCl₃, or BCl₃ and washing with NaOH to set as a DNA adsorbant; United States Patent: 5,616,701: DNA purification by solid phase extraction using a hydroxide-washed glass fiber membrane; United States Patent: 5,650,506: Modified glass fiber membranes useful for DNA purification by solid phase extraction).

Bedingung für diese Nukleinsäurebindung ist dabei immer, dass die für die Bindung verwendeten Membranen durch chemische Modifizierungsreaktionen mit positiven Ionenladungen dotiert werden. Damit liegt auf der Hand, das es zwischen der positiv geladenen Oberfläche der eingesetzten Membranen und der negativen Ionenladung des Phosphatrückgrats von Nukleinsäuren durch Coulombsche Wechselwirkungen eine Bindung ergeben wird. Insofern wird das der Fachwelt hinlänglich bekannte Prinzip der Bindung von Nukleinsäuren an positive geladene feste Phasen genutzt, welches bekanntermaßen z.B. für DNA/RNA-Blotting-Techniken an positiv geladenen Nylon-Filtern schon viele Jahre eine Standardapplikation darstellt.

Ein ganz wesentlicher Nachteil dieser beschriebenen Verfahren besteht allerdings darin, dass diese nicht zur Nukleinsäureisolierung geeignet sind, d.h. es ist komplett unmöglich Nukleinsäuren aus komplexen Ausgangsmaterialien zu isolieren. Ausgangsmaterial sind immer schon bereits isolierte Nukleinsäuren, welche wie in den zitierten US-Patentschriften dargestellt, in an sich bekannter Weise isoliert werden müssen. Insbesondere ein Aspekt erscheint dem Fachmann dabei unklar. Die beschriebenen Bindungsbedingungen (Bindung unter physiologischen Pufferbedingungen) und Elutionsbedingungen sind identisch. Es ist nicht zu ersehen wie unter denselben Pufferbedingungen zur Bindung der Nukleinsäuren an die positiv geladene Membran auch wieder die Nukleinsäuren von der Membran abgelöst werden können.
Letztlich können die dargestellten Mittel und das dazugehörende Verfahren eine nur sehr enge praktische Anwendung besitzen. Bekannt sind auch synthetisch hergestellte Oligonukleotide an die positiven Oberflächen zu binden. Dies erfolgt dabei wiederum unter Ausnutzung Coulombscher Wechselwirkungen, d.h. auf der Basis der Verknüpfung positiver und negativen Ladungen z.B. über modifizierte Oligonukleotide (Verknüpfung mit Aminolinkern oder Phospatlinkern). Auch diese Varianten ermöglichen nicht die Isolierung von Nukleinsäuren aus komplexen Ausgangsmaterialien.

Wie ausführlich dargestellt, existieren alternative Formen der Nukleinsäurebindung zur Reinigung an Membranen mit ausreichender positiver Ladung, die keine Verfahren zur Isolierung von Nukleinsäuren darstellen. Die Bindung der Nukleinsäuren erfolgt durch Coulombsche Kräfte, basierend auf Wechselwirkungen zwischen positiven Ionenladungen der Membranen und der negativen Ionenladungen des Nukleinsäurenrückrats. Dieses Prinzip erscheint damit logisch erklärbar.

Basierend auf der erfindungsgemäßen Isolierung von Nukleinsäuren aus komplexen Ausgangsmaterialien mit antichaotropen Salze wurde folgendes gefunden. So zeigte sich, dass sich auch negativ geladene Oberfläche oder Oberflächen, die in ein negatives Ladungspotential überführt werden können, für die Bindung von Nukleinsäuren unter Verwendung der erfindungsgemäßen Lyse/Bindungspuffersysteme eignen.
Die erfindungsgemäß eingesetzten negativ funktionalisierten Oberflächen oder mit potientiell negativen Modifizierungen ausgestattete Oberflächen werden nach an sich bekannten Verfahren erzeugt. Als geeignet hat sich z.B. die photochemische Kopplung einer Acetylgruppe, Carboxylgruppe oder Hydroxylgruppe an die Oberfläche eines Reaktionsgefäßes gezeigt.

Mit der vorliegenden Verfahrensvariante ermöglichen sich völlig neue Perspektiven für eine komplexe Nukleinsäureanalytik. Es zeigte sich nämlich, dass für die Bindung der Nukleinsäure an negative oder potentiell negative Oberflächen die Nukleinsäure wie bei allen bisher beschriebenen Varianten nicht schon isoliert sein muss. Die Bindung erfolgt aus dem Lysereaktionsansatz heraus, d.h. die die Nukleinsäure enthaltende Ausgangsprobe wird lysiert und die frei werdenden Nukleinsäuren binden an die negativ geladene Oberfläche (z.B. an eine Mikrotestplattenkavität oder ein Reaktionsgefäß).

Durch die erfindungsgemäße Verfahrensvariante können nunmehr völlig neuartige "Single Tube" und Einschritt-Verfahren zur Isolierung von Nukleinsäuren aus komplexen Ausgangsmaterialien realisiert werden. Solche Verfahren bieten in ihrem Anwendungsspektrum für die Nutzer riesige Vorteile (Einfachheit; Billigkeit; Reduzierung von Abfall, Schnelligkeit, Routinetauglichkeit, Automatisierbarkeit u.a.m.)

Eine weitere Anwendung dieser Verfahrensvariante besteht außerdem darin, nicht nur die Extraktion der Nukleinsäuren in einer Reaktionskavität zu realisieren, sondern auch eine folgende Targetamplifikation und ggf. nachfolgende Analytik im selben Reaktionsgefäß durchzuführen, ggf. Hybridisierungsreaktionen durchzuführen oder Sequenzierungen an festen Phasen ablaufen zu lassen.

Auf dieser Basis wird z.B. ein 0.5 ml PCR-Reaktionsgefäß mittels der Fachwelt bekannter Techniken mit einer negativ geladenen oder potentiell negativen funktionalen Gruppe modifiziert. Dazu eignet sich z.B. die photochemische Kopplung einer Acetylgruppe, Carboxylgruppe oder Hydroxylgruppe an die Oberfläche des Reaktionsgefäßes. In das Reaktionsgefäß wird dann die für die Nukleinsäurenisolierung ausgewählte Probe gegeben (z.B. Vollblut) und mit einem Lysepuffer, enthaltend die antichaotrope Salzfraktion z.B. Ammoniumchlorid, ein Detergenz und ein proteolytisches Enzym versetzt und das Gefäß bei 70°C für 5 min. inkubiert.

Zur Maximierung der Nukleinsäurenbindung kann nach der Lyse des Ausgangsmaterials noch ein Detergenz/Alkohol-Gemisch pipettiert werden. Der Ansatz wird dann für kurz inkubiert und nachfolgend aus dem Reaktionsgefäß abgegossen. Die Nukleinsäure befindet sich nun an der funktionalisierten Oberfläche des Reaktionsgefäßes gebunden und wird nachfolgend mit einem alkoholischen Waschpuffer kurz gespült und der Alkohol wird durch Inkubation bei z.B. 70°C entfernt. Die Elution der gebundenen Nukleinsäuren erfolgt weiter fachgemäß durch die Zugabe eines Niedrigsalzpuffers (z.B. 10 mM Tris-HCl) in das Reaktionsgefäß und eine kurze Inkubation (z.B. 2 min) bei z.B. 70°C. Die Nukleinsäure ist so für nachfolgende Verwendungen verfügbar.

Wie dargestellt laufen alle Reaktionen der Nukleinsäureisolierung aus einem komplexen Ausgangsmaterial in einem Reaktionsgefäß ab; d.h. Lyse des Ausgangsmaterials, Bindung der Nukleinsäuren; Waschen der gebundenen Nukleinsäuren und Elution der Nukleinsäuren werden in und mit einem Reaktionsgefäß realisiert.

Die den momentan weltweit am Häufigsten genutzten Extraktionskits der Fa. Qiagen benötigen für die Abfolge von Lyse, Bindung, Waschen und Elution jeweils eine Filterkartusche und mindestens 4 separate Reaktionsgefäße eingeschlossen sind des weiteren multiple Zentrifugationsschritte.

Das erfindungsgemäße Verfahrensvariante erlaubt im Gegensatz dazu die Extraktion der Nukleinsäure ohne einen einzigen Zentrifugationsschritt. Daraus läßt sich auch ein enormer zeitlicher Vorteil ableiten. Diese Vorteile beziehen sich auch auf die beschriebenen Nukleinsäureextraktionsverfahren des zitierten US 5,234,809 von Boom.

Neben der möglichen Extraktion von Nukleinsäure kann die gebundene Nukleinsäure aber auch an der Oberfläche des beschriebenen 0.5 ml Reaktionsgefäßes verbleiben und z.B. nachfolgend durch Zugabe eines kompletten PCR-Raktionsmixes (Primer, Nukleotide, Polymerasepuffer, Taq Polymerase, Magnesium) gleich für eine PCR-Applikation verwendet werden, d.h. Extraktion und Amplifikation laufen dann im selben Reaktionsgefäß ab.

Diese Beispiele illustrieren die enormen Vorteile und breite Anwendbarkeit, die aus der Erfindung ableitbar sind. Sie ermöglicht in einer Ausführungsvariante den kompletten Vorgang von Probenvorbereitung über Amplifikation und ggf. auch Analytik in z.B. einer Reaktionskavität. Damit ergeben sich mit der Bereitstellung von modifizierten Reaktionsgefäßen (oder auch anderen festen Oberflächen) und den geeigneten Lyse/Bindungspuffern neue Standards in molekularbiologisch und vor allem Nukleinsäure-Diagnostik bearbeitenden Laboratorien, wobei durch die neuen potentiellen applikativen Lösungen vor allem auch die hinlänglich bekannten Probleme der Probenkontaminationen drastisch reduziert werden.

Ein weiterer Vorteil und auch eine weitere Applikation besteht darin, dass die oberflächenfixierten Nukleinsäuren an der Oberfläche auch zumindest längere Zeit stabil fixiert und somit für eine spätere Bearbeitung verfügbar sind, d.h die PCR-Reaktion muss sich nicht zwingend sofort der Extraktion anschließen. Ein weiteres Anwendungsfeld ist die vollautomatisierte Nukleinsäureextraktion und ggf. Analytik unter Verwendung der hier beschriebenen mit negativen oder mit potentiell negative Ladungen tragenden Oberflächen, vorzugsweise Plastikoberflächen geeigneter Reaktionskavitäten z.B. Mikrotestplatten).

Die erfindungsgemäßen Lyse/Bindungspuffersysteme mit den antichaotropen Salzen als Hauptkomponenten einschließlich ggf. eines proteolytischen Enzyms können auch als feste Formulierung bereitgestellt. Dazu werden die Mischungen aus Salzen und Detergenzien, Zusatzstoffen und ggf. Enzymen in gebräuchlichen Rektionsgefäßen aliquotiert und für mehrere Stunden bei 95 °C inkubiert oder nach an sich bekannten Verfahren lyophilisiert und so in eine feste Formulierung überführt.

Diese feste Formulierungen in fertigen komplexen Reaktionsmixen für die Nukleinsäureisolierung sind langzeitlagerstabil, d.h. auch die biologische Aktivität der proteolytischen Enzymkomponente bleibt bei einer Langzeitlagerung (siehe Ausführungsbeispiel) bestehen. Die Herstellung der festen Formulierung von Lysepuffermixen erfolgte dabei ohne den Zusatz von an sich bekannten protektiven Zusatzstoffen, einfach durch eine Kältelyophilisierung.

Alle kommerziell angebotenen Testkits zur Nukleinsäurenextraktion enthalten die notwendigen Komponenten einzeln, bestimmte Lösungen müssen durch den Anwender erst hergestellt werden und darüber hinaus sind die Lösungen in ihrer Haltbarkeit eingeschränkt. Ein weiterer Nachteil besteht darin, dass der Nutzer während der Isolierung von Nukleinsäuren unter Verwendung z.Z. gebräuchlicher Testkits multiple Pipettierschritte für verschiedene Einzellösungen einhalten muss. Dies erhöht vor allem im Bereich der medizinischen Diagnostik das Kontaminationsrisiko dramatisch. Nachteilig ist ferner auch, dass durch die z.B. existierenden Beladungsgrenzen von weit gebräuchlichen Zentrifugationssäulchen, welche hauptsächlich für die Nukleinsäureisolierung angewendet werden, auch die Menge des Ausgangsmaterials stark begrenzt ist. Dies liegt darin begründet, das zum Ausgangsmaterial noch die für die Extraktion notwendigen Lyse- und Bindungspuffer zugegeben werden müssen.

Durch die Bereitstellung einer festen Formulierung als lagerstabiler Lysemix auf der Basis antichaotroper Salze werden alle die bestehenden Probleme in ganz einfacher Form gelöst.

Diese Formulierung hat folgende Vorteile:
1. Langzeitlagerung von. "Ready to use" Lysepuffermixen
2. Stabilisierung von proteolytischen Enzymen in fertigen Lysemixturen und deren Langzeitlagerung
3. Einsatz von größeren Mengen an Ausgangsmaterialien bei gleicher Dimensionierung von bestehenden Zentrifugationssäulchen (z.B. Verdreifachung der Ausgangsmenge)
4. Reduzierung von Kontaminationsrisiken durch die Reduzierung von Pipettierschritten und Lösungen
5. Probenaufnahme im fertigen Lysemix auch außerhalb des Labors und deren ggf. Langzeitlagerung
6. stabiler Probenversand und Kühlung

Die fertigen festen, stabilen Lysepuffermixe bestehend aus einer Vielzahl von Einzelkomponenten; einschließlich ggf. proteolytischen Enzymen sind einfach handhabbar (auch von Personen ohne Sachkenntnisse), da die Reaktion einfach durch Zugabe einer Probe, die die zu isolierende Nukleinsäure enthält, gestartet wird. Darüber hinaus kann davon ausgegangen werden, dass die Mixturen entsprechend ihrer Inhaltstoffe Haltbarkeiten von mindestens 6 Monaten aufweisen, wodurch auch ein Transport der Probe bei Umgebungstemperatur kein Problem mehr darstellt.

Der Vorteil der festen Formulierungen basiert darauf, dass für die Lyse von Nukleinsäuren (NAs) enthaltenden Probenmaterialien eine diese NAs enthaltende Probe lediglich in das Reaktionsgefäß mit dem enthaltenden lagerstabilen Lysepuffer überführt wird und ggf. durch die Zugabe von Wasser, die Probe im jeweiligen Reaktionsgefäß lysiert wird. Aufwendige und kontaminationsbelastende multiple Pipettierschritte entfallen gänzlich. Vor allem für die Sammlung und Aufarbeitung von klinischen und forensischen Proben unter Feldbedingungen sind durch die erfindungsgemäße Formulierung die bekannten Probleme gelöst und es steht eine einfach zu handhabende Formulierung zur Verfügung.

Überaschenderweise zeigte sich dann ebenfalls in der praktischen Durchführung, daß nach Zugabe des zu lysierenden Ausgangsmaterials ggf. bei Zugabe einer festen Probe nach Zugabe von H₂O die feste Formulierung unter Standardreaktionsbedingungen problemlos wieder in eine flüssige Phase überführbar ist.

Das Lyse/Bindungspuffersystem kann als wässerige Lösung vorliegen oder als feste Formulierung in einsatzfertigen Reaktionsgefäßen.
Als feste Phase können alle Trägermaterialien fungieren, die zur Isolierung mittels chaotroper Reagentien Anwendung finden, vorzugsweise Glasfaservliese, Glasmembranen, Siliciumträger und Aerosile oder Trägermaterialien, die eine negativ geladene Oberfläche besitzen oder chemisch modifizierte Oberflächen aufweisen, die in ein negatives Ladungspotential besitzen.

Gegenstand der Erfindung ist ferner ein Verfahren zur Isolierung von Nukleinsäuren, insbesondere von DNA, aus beliebigen komplexen Ausgangsmaterialien unter Verwendung der genannten Formulierungen, das durch Lyse des Ausgangsmaterial, Bindung der Nukleinsäuren an ein Trägermaterial, Waschung der am Träger gebundenen Nukleinsäuren und Elution der Nukleinsäuren gekennzeichnet ist.

Aufgrund der erzielten DNA-Qualität ist es auch zur präparativen Isolierung und Aufreinigung für DNA zum Einsatz in der Gentherapie gut geeignet.

Gegenstand der Erfindung sind auch lagerstabile und einsatzfertige feste Formulierung von Lysepuffersystemen für die Nukleinsäureisolierung auf der Basis antichaotroper Salzen, die als "Ready-to-Use"-Mixe einsatzfertig in konventionellen Reaktionsgefäßen vorliegen. Die festen Formulierungen, der Lysepufferansätze werden durch die Zugabe von lediglich der Probe (bei flüssigen Proben wie z.B. Vollblut, Speichel, Zellsuspensionen, Serum, Plasma, Liquor), bei festen Ausgangsmaterialien wie Gewebe, Haarwurzeln, Blutspuren an festen Oberflächen, Zigarettenkippen deparaffinierte Gewebe u.a.m. zusätzlich durch Zugabe von Wasser aktiviert und realisieren die Lyse des Ausgangsmaterials. Nach erfolgter Lyse des Ausgangsmaterials wird der Lyseansatz in an sich bekannter Weise ggf. nach Zugabe einer ethanolische Lösung bzw. eines Alkohol/Detergenz-Gemisches mit den Verwendung findenden nukleinsäurebindenden festen Phasen jeglicher Form (Suspension, Zentrifugationssäulchen) inkubiert. Die nachfolgende Bindung der Nukleinsäuren an die jeweiligen festen Phasen, das Waschen der gebundenen Nukleinsäuren und die finale Elution erfolgen wie schon beschrieben nach dem Stand der Technik.
Mit diesen festen Formulierungen sind neuartige Lösungen vor allem für die Anwendungsgebiete jeglicher Form von Nukleinsäurendiagnostik gegeben.

Hervorgehoben werden soll noch einmal, dass die Erfindungsvariante in einem Einschritt-Verfahren und in einem "Single Tube"-Verfahren die Isolierung von Nukleinsäuren aus komplexen Ausgangsmaterialien, ggf. Targetamplifikationen und ggf. nachfolgende Analytik des amplifizierten Nukleinsäureabschnittes ermöglicht. Ausgangsmaterial muss dabei nicht eine schon isolierte Nukleinsäure sein, sondern ist das komplexe die Nukleinsäure enthaltende Ausgangsmaterial. Die für die Bindung der Nukleinsäure benötigte Oberfläche enthält negative oder potentiell negative funktionale Gruppen. Die Bindung der Nukleinsäure wird in einem Lyse/Bindungspuffer realisiert, wobei die für die Bindung der negativ geladenen Nukleinsäure an die negative funktionalisierte Oberfläche benötigten Ionen aus antichaotropen Salzen stammen.
Damit sind realisierbar:
1. "Single Tube"-Verfahren zur Isolierung von Nukleinsäuren aus komplexen Ausgangsmaterialien
2. "Single Tube"-Verfahren zur Isolierung von Nukleinsäuren aus komplexen Ausgangsmaterialien und nachfolgende Targetvervielfältigung
3. "Single Tube"-Verfahren zur Isolierung von Nukleinsäuren aus komplexen Ausgangsmaterialien, nachfolgende Targetvervielfältigung und nachfolgende Analytik des vervielfältigten Nukleinsäureabschnittes.

Das bedeutet sowohl Nukleinsäure-Isolierung aus unterschiedlichsten DNA enthaltenen Ausgangsmaterialien ggf. Targetvervielfältigung und ggf. Analytik finden in ein und der selben Reaktionskavität oder ggf. auf ein und der selben Reaktionsoberfläche statt.

Die erfindungsgemäßen Formulierungen und das universelle Verfahren zur Bindung von Nukleinsäuren an festen Phasen zur Isolierung, Aufreinigung und nachfolgenden komplexen molekularen Analytik von Nukleinsäuren aus beliebigen Ausgangsmaterialien und Mengen, welche Nukleinsäuren enthalten, stellen eine neuartige Plattformtechnologie für die Entwicklung von integrativen vollautomatisierbaren genanalytischen Systemen dar, die es ermöglichen, Probenvorbereitung, Targetvervielfälltigung und Targetanalytik in einer Reaktionskavität zu realisieren.

Die Erfindung wird anschließend an einem Ausführungsbeispiel näher erläutert.

### Ausführungsbeispiel:

### Bindung von DNA an eine Festphase

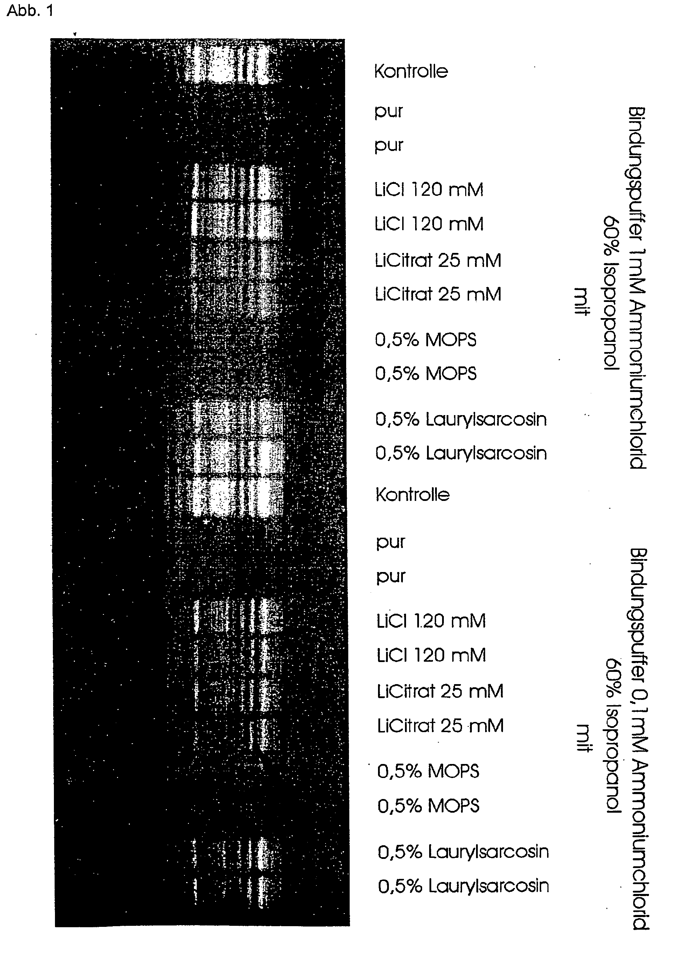

1µg eines DNA-Längenstandards (GeneRuler DNA Ladder Mix, Fermentas) wurde in einem Puffer, bestehend aus in der Abbildung dargestellten Komponenten auf eine Zentrifugationssäule mit einer Glasfasermembran (Micro Spin Säule, Safeclick) überführt. Danach folgte eine Zentrifugation für 2 min bei 12.000 rpm und Verwerfen des Filtrates. Nach Trocknung durch einen kurzen Zentrifugationsschritt (12.000 rpm für 2min) erfolgte die Zugabe von 10µl eines Elutionspuffers (10 mM Tris-HCl; pH 8,0) und Elution der DNA durch Zentrifugation für 1 min bei 10.000 rpm.
Jeweils 10µl der eluierten DNA wurden anschließend auf ein Agarosegel geladen und nach Ethidiumbromidfärbung dargestellt (Abbildung 1).

## Patentansprüche

1. Formulierungen ohne chaotrope Bestandteile zur Isolierung von Nukleinsäuren unter Bindung an eine feste Phase, insbesondere von DNA aus beliebigen komplexen Ausgangsmaterialien enthaltend
- ein Lyse/Bindungspuffersystem, das mindestens eine antichaotrope Salzkomponente aufweist,
- eine feste Phase,
- Wasch- und Elutionspuffer,
**dadurch gekennzeichnet, dass** die Konzentration der antichaotropen Salzkomponenten zwischen 0,1 mM und 1 mM beträgt.

2. Formulierungen nach Anspruch 1, **dadurch gekennzeichnet, dass** Detergenzien und Zusatzstoffe Tris-HCl, EDTA, Polyvinylpyrrolidone, CTAB, TritonX-100, N-Lauryl-Sarkosin, Natriumcitrat, DTT, SDS und/oder Tween sind.

3. Formulierungen nach Anspruch 1 bis 2, **dadurch gekennzeichnet, dass** das Lyse/Bindungspuffersystem zur Bindung an die feste Phase einen Alkohol aufweist.

4. Formulierungen nach Anspruch 1 bis 3, **dadurch gekennzeichnet, dass** das Lyse/Bindungspuffersystem Enzyme, vorzugsweise Protein-abbauende Enzyme, aufweist.

5. Formulierungen nach Anspruch 1 bis 4, **dadurch gekennzeichnet, dass** das Lyse/Bindungspuffersystem als feste lagerstabile Formulierung in einsatzfertigen Reaktionsgefäßen vorliegt.

6. Formulierungen nach Anspruch 1 bis 5, **dadurch gekennzeichnet, dass** als feste Phase Glasfaservliese, Glasmembranen, Gläser, Zeolithe, Keramik oder Siliciumträger fungieren.

7. Formulierungen nach Anspruch 1 bis 6 , **dadurch gekennzeichnet, dass** als feste Phase Trägermaterialien fungieren, die eine negativ funktionalisierte Oberfläche besitzen oder funktionalisierte Oberflächen aufweisen, die in ein negatives Ladungspotential überführt werden können.

8. Verfahren zur Isolierung von Nukleinsäuren, insbesondere von DNA, aus beliebigen komplexen Ausgangsmaterialien unter Verwendung von Formulierungen nach einem der Ansprüche 1 bis 7, **dadurch gekennzeichnet, dass** das Ausgangsmaterial lysiert wird, die Bindung der Nukleinsäuren an eine feste Phase erfolgt, die am Träger gebundenen Nukleinsäuren gewaschen werden und die Elution der Nukleinsäuren erfolgt.

9. Verfahren zur Isolierung von Nukleinsäuren nach Anspruch 8, **dadurch gekennzeichnet, dass** man das DNA enthaltende Material mit einem
- Lyse/Bindungspuffersystem, umfassend eine wässerige Lösung, die eine antichaotrope Salzkomponente, mindestens ein Detergenz, ggf. Zusatzstoffe sowie ggf. ein proteolytischen Enzym enthält, und
- mit einer festen Phase ggf. unter Zusatz eines Alkohols in Kontakt bringt,
- anschließend wäscht und die Nukleinsäure von der festen Phase löst.

10. Verfahren zur Isolierung von Nukleinsäuren, insbesondere von DNA, aus beliebigen komplexen Ausgangsmaterialien unter Verwendung von Formulierungen nach einem der Ansprüche 1 bis 8, **dadurch gekennzeichnet, dass**
- das Ausgangsmaterial in einem "Single Tube"- bzw. Einschritt-Verfahren mit einer negativ funktionalisierten Oberfläche oder dessen Oberfläche chemisch so modifiziert ist, dass sie in ein negatives Ladungspotential überführt werden kann, in Kontakt gebracht und lysiert wird ,
- die Bindung der Nukleinsäure an die Oberfläche erfolgt,
- die gebundene Nukleinsäure gewaschen und ggf. elutiert wird.

11. Verfahren nach Anspruch 9 bis 10, **dadurch gekennzeichnet, dass** negativ funktionalisierte Oberflächen entsprechend modifizierte planare Oberflächen, Filtermembranen, herkömmliche Plastikgefäße oder Mikrotestplatten sind.

12. Verfahren nach Anspruch 10 bis 11, **dadurch gekennzeichnet, dass** die Nukleinsäure anschließend im gleichen Reaktionsansatz einer Amplifikationsreaktion ausgewählter Sequenzabschnitte unterzogen und ggf. daran anschließend eine Analyse der Gensequenzen durchgeführt wird.

13. Verfahren nach Anspruch 10 bis 12, **dadurch gekennzeichnet, dass** die Nukleinsäure anschließend im gleichen Reaktionsansatz hybridisiert oder sequenziert wird.

14. Verwendung antichaotroper Komponenten in einem Lyse/Bindungspuffersystem gemäß Anspruch 1 zur Isolierung und Reinigung von Nukleinsäuren unter Bindung an eine feste Phase.

15. Verwendung nach Anspruch 14, **dadurch gekennzeichnet, dass** das Lyse/Bindungspuffersystem als feste lagerstabile Formulierung vorliegt.

## Claims

1. Formulations without chaotropic components for the isolation of nucleic acids with binding to a solid phase, in particular of DNA from arbitrary complex starting materials, containing
- a lysis/binding buffer system manifesting at least one anti-chaotropic salt component,
- a solid phase,
- washing and elution buffers,
wherein the concentration of the anti-chaotropic salt components is between 0.1 mM and 1 mM.

2. Formulations according to Claim 1, wherein detergents and additives are Tris-HCl, EDTA, polyvinylpyrrolidone, CTAB, TritonX-100, N-Lauryl-Sarcosine, sodium citrate, DTT, SDS and/or Tween.

3. Formulations according to one of the Claims 1 or 2, wherein the lysis/binding buffer system manifests an alcohol for binding on to the solid phase.

4. Formulations according to one of the Claims 1 to 3, wherein the lysis/binding buffer system manifests enzymes, preferably protein-decomposing enzymes.

5. Formulations according to one of the Claims 1 to 4, wherein the lysis/binding buffer system is available as a solid, storage-stable formulation in ready-to-use reaction vessels.

6. Formulations according to one of the Claims 1 to 5, wherein preferably glass fibre fleeces, glass membranes, glasses, zeoliths, ceramics or silicone carriers function as solid phase,.

7. Formulations according to one of the Claims 1 to 6, wherein carrier materials possessing a negatively functionalised surface or manifesting functionalised surfaces which can be converted to a negative charging potential function as a solid phase.

8. Method for the isolation of nucleic acids, in particular of DNA, from arbitrary complex starting materials making use of formulations according to one of the Claims 1 to 7, wherein the starting material is lysed, the binding of the nucleic acids to a solid phase takes place, the nucleic acids bound to the carrier are washed and the elution of the nucleic acids takes place.

9. Method for the isolation of nucleic acids according to Claim 8, wherein the material containing the DNA
- is brought into contact with a lysis/binding buffer system entailing an aqueous solution containing an anti-chaotropic salt component, at least one detergent, if need be additives and if need be a proteolytic enzyme, and
- with a solid phase, if need be making use of an alcohol,
- is then washed and the nucleic acid dissolved from the solid phase.

10. Method for the isolation of nucleic acids, in particular of DNA, from arbitrary complex starting materials making use of formulations according to one of the Claims 1 to 8, wherein
- the starting material is brought into contact with a negatively functionalised surface or with a surface which has been chemically modified in such a way that it can be converted into a negative charge potential in a single-tube or a single-step process and is lysed,
- the nucleic acid is bound to this surface,
- the bound nucleic acid is washed and eluted if necessary.

11. Method according to Claims 9 to 10, wherein negatively functionalised surfaces are correspondingly modified planar surfaces, filter membranes, conventional plastic vessels or micro-test plates.

12. Method according to Claims 10 to 11, wherein the nucleic acid is subsequently subjected to an amplification reaction of selected sequence sections in the same reaction mixture and then, if need be, an analysis of the genetic sequences is carried out.

13. Method according to Claims 10 to 12, wherein the nucleic acid is then hybridised or sequenced in the same reaction mixture.

14. Use of anti-chaotropic components in a lysis/binding buffer system according to Claim 1 for isolation and purification of nucleic acids with binding to a solid phase.

15. Use according to Claim 14, wherein the lysis/binding buffer system is available as a solid, storage-stable formulation.

## Revendications

1. Formulations sans éléments chaotropiques, destinées à l'isolation des acides nucléiques en les liant à une phase solide, en particulier de l'ADN extrait de matières de départ complexes quelconques, contenant
- un système tampon de lyse/liaison qui présente au moins un composant de sel antichaotropique,
- une phase solide,
- un tampon de lavage et d'élution,
**se caractérisant par** une concentration des composants de sel antichaotropiques entre 0,1 mM et 1 mM.

2. Formulations selon la revendication 1, **se caractérisant par le fait que** les détergents et additifs sont des Tris-HCl, EDTA, pyrrolidones polyvinyliques, CTAB (bromure d'hexadécyltriméthylammonium), TritonX-100, N-lauryl sarcosine, citrate de sodium, DTT (dithiothréitol), SDS (dodécylsulfate de sodium) et/ou Tween.

3. Formulations selon les revendications 1 à 2, **se caractérisant par le fait que** le système tampon de lyse/liaison présente un alcool pour la liaison à la phase solide.

4. Formulations selon les revendications 1 à 3, **se caractérisant par le fait que** le système tampon de lyse/liaison présente des enzymes, de préférence des enzymes qui décomposent les protéines.

5. Formulations selon les revendications 1 à 4, **se caractérisant par le fait que** le système tampon de lyse/liaison existe sous forme de formulation solide, stable à la conservation dans des récipients à réaction prêts à l'emploi.

6. Formulations selon les revendications 1 à 5, **se caractérisant par le fait que** des non-tissés de fibre de verre, des membranes de verre, des verres, des zéolithes, des céramiques ou des supports en silicium jouent le rôle de phase solide.

7. Formulations selon les revendications 1 à 6, **se caractérisant par le fait que** des matières supports qui possèdent une surface fonctionnalisée, chargée négativement, ou présentent des surfaces fonctionnalisées qui peuvent être transférées dans un potentiel de charge négatif, jouent le rôle de phase solide.

8. Procédé destiné à l'isolation d'acides nucléiques, en particulier de l'ADN, extrait de matières de départ complexes quelconques en utilisant des formulations selon l'une des revendications 1 à 7, **se caractérisant par le fait que** la matière de départ sera lysée, que les acides nucléiques se lient à une phase solide, que les acides nucléiques liés au support seront lavés et que les acides nucléiques seront élués.

9. Procédé destiné à l'isolation d'acides nucléiques selon la revendication 8, **se caractérisant par le fait que** l'on met en contact la matière contenant l'ADN
- avec un système tampon de lyse/liaison, englobant une solution aqueuse qui contient un composant de sel antichaotropique, au moins un détergent, le cas échéant des additifs ainsi que le cas échéant une enzyme protéolytique, et
- avec une phase solide, le cas échéant en additionnant un alcool,
- qu'on la lave ensuite et que l'on sépare l'acide nucléique de la phase solide.

10. Procédé destiné à l'isolation d'acides nucléiques, en particulier de l'ADN, extrait de matières de départ complexes quelconques en utilisant les formulations selon l'une des revendications 1 à 8, **se caractérisant par le fait que**
- la matière de départ est modifiée chimiquement au cours d'un procédé "Single Tube" ou de coupure, avec une surface fonctionnalisée, chargée négativement, ou dont la surface est modifiée de sorte qu'elle puisse être transférée dans un potentiel de charge négatif, est mise en contact et lysée,
- l'acide nucléique se lie à la surface,
- l'acide nucléique lié est lavé et élué le cas échéant.

11. Procédé selon les revendications 9 à 10, **se caractérisant par le fait que** les surfaces fonctionnalisées, chargées négativement, sont en conséquence des surfaces planaires modifiées, des membranes filtrantes, des récipients en plastique usuels ou des plaques microtest.

12. Procédé selon les revendications 10 à 11, **se caractérisant par le fait que** l'acide nucléique est ensuite, dans le même mélange réactionnel, soumis à une réaction d'amplification de sections de séquence sélectionnées et que le cas échéant une analyse des séquences génétiques est réalisée à la suite.

13. Procédé selon les revendications 10 à 12, **se caractérisant par le fait que** l'acide nucléique est hybridisé ou séquencé ensuite dans le même mélange réactionnel.

14. Emploi de composants antichaotropiques dans un système tampon de lyse/liaison conformément à la revendication 1 pour isoler et purifier des acides nucléiques en les liant à une phase solide.

15. Emploi selon la revendication 14, **se caractérisant par le fait que** le système tampon de lyse/liaison existe sous forme de formulation solide stable à la conservation.
